# EUROPEAN PATENT APPLICATION

(11) **EP 4 748 335 A1**
(43) Date of publication of application: **27.05.2026**
(21) Application number: 24842338.6
(22) Date of filing: 15.07.2024
(51) Int. Cl.: A61B 34/30

(54) **CLAMPING INSTRUMENT TAIL END, ENERGY INSTRUMENT TAIL END, AND SURGICAL INSTRUMENT**

(30) Priority: 19.07.2023 CN 202310891156; 19.07.2023 CN 202310890935
(71) Applicant: Shanghai Microport Medbot (Group) Co., Ltd., Shanghai 201203 (CN)
(72) Inventor: HE, Yuyuan, Shanghai 201203 (CN); HE, Chao, Shanghai 201203 (CN)
(74) Representative: Patentship Patentanwaltsgesellschaft mbH
(86) International application number: PCT/CN2024/105391
(87) International publication number: WO 2025/016346

(57) **Abstract**

A clamping instrument tail end, an energy instrument tail end, and a surgical instrument. The clamping instrument tail end comprises a pitch base body (1000) and a swing base body (2000). A distal end surface of the pitch base body (1000) is provided with a first contact boss (1100) protruding from the distal end surface; the pitch base body (1000) is provided with first traction channels (1200), and channel distal end ports of the first traction channels (1200) are located on the distal end surface of the pitch base body (1000); a proximal end surface of the swing base body (2000) is provided with a second contact boss (2100) protruding from the proximal end surface; the swing base body (2000) is rotatably fitted to the pitch base body (1000), so that the first contact boss (1100) is in rolling contact with the second contact boss (2100); and the swing base body (2000) is provided with guide channels (2200), and channel proximal end ports of the guide channels (2200) are located on the proximal end surface of the swing base body (2000). The deflection angle (β) of a first traction body (4100) is less than the pivot angle (α) of the relative rotation between the swing base body (2000) and the pitch base body (1000), thereby ameliorating the bending radius of the first traction body (4100), and reducing the wear of the first traction body (4100). An energy release body (3100) is provided on the swing base body (2000), and an energy transmission cable (3200) passes through the first traction channel (1200) and the guide channel (2200) to be connected to the energy release body (3100). A position where the energy transmission cable (3200) passing through the guide channel (2200) is bent is located at the proximal end surface of the swing base body (2000), so that the bending radius of the energy transmission cable (3200) can be ameliorated.

## Description

### TECHNICAL FIELD

This application relates to the field of medical devices and, in particular, to a clamping instrument tail end, an energy instrument tail end and a surgical instrument.

### BACKGROUND

With the ever-increasing use and advancement of robotics-related technologies, especially the development of computing technology, more and more importance is being attached to clinical use of medical surgical robots. Minimally invasive surgical robot systems apply interventional therapy, which involves less physical labor of surgeons during surgery, enables precise surgery and allows for reduced trauma, less blood loss, fewer postoperative infections and faster recovery of patients. The design of a surgical instrument used in a minimally invasive surgical robot system directly determines whether a surgical procedure performed using the minimally invasive surgical robot system can succeed or not. Well-designed surgical instruments can better assist surgeons in performing surgical operations. Therefore, the performance of a surgical instrument is considered critical to the performance of a minimally invasive surgical robot system in which it is employed.

A surgical instrument designed to clamp something is required to make good pitch and yaw motions, which can eventually effect precise clamping actions. However, for existing surgical instruments, a large angle of pitch means a large winding angle of a traction such as a steel wire cable around a guide pulley. If such winding of the steel wire cable around the guide pulley takes place frequently at large winding angles, the service life of the cable tends to be shortened.

Energy instruments with multiple degrees of freedom, such as electrosurgical hooks, electrosurgical spatulas or bipolar electrosurgical forceps, are prone to cable flexure during various movements, which may cause accidents such as cable jacket damage and weld or joint detachment, leading to unreliable insulation and a shortened lifespan.

### SUMMARY

In view of this, there is a need for a clamping instrument tail end, an energy instrument tail end and a surgical instrument, which overcome the problems described above.

A clamping instrument tail end disclosed herein is including:
a pitch base body provided on a distal end face thereof with a first contact boss protruding from the distal end face, the pitch base body defining first traction channels each having a distal opening located at the distal end face of the pitch base body; and
a swing base body provided on a proximal end face thereof with a second contact boss protruding from the proximal end face, the swing base body pivotably assembled with the pitch base body so that the first contact boss is in rolling contact with the second contact boss, the swing base body defining guide channels each having a proximal opening located at the proximal end face of the swing base body.

### BRIEF DESCRIPTION OF THE DRAWINGS

Fig. 1 shows a perspective view of a clamping instrument tail end according to an embodiment disclosed herein.
Fig. 2 shows an exploded view of the clamping instrument tail end according to the embodiment.
Fig. 3 shows the clamping instrument tail end according to the embodiment during pivoting.
Fig. 4 shows a half cutaway view of the clamping instrument tail end according to the embodiment in a straight configuration.
Fig. 5 shows a partial cutaway view of the clamping instrument tail end according to the embodiment in the straight configuration.
Fig. 6 shows a half cutaway view of the clamping instrument tail end according to the embodiment during pivoting.
Fig. 7 shows a partial cutaway view of the clamping instrument tail end according to the embodiment during pivoting.
Fig. 8 shows a perspective view of a pitch base body according to the embodiment.
Fig. 9 shows a perspective cutaway view of a pitch base body according to the embodiment.
Fig. 10 shows a top view of the pitch base body according to the embodiment.
Fig. 11 shows a perspective view of the swing base body according to the embodiment.
Fig. 12 shows a perspective side view of the swing base body according to the embodiment.
Figs. 13 and 14 show how the swing base body pivots relative to the pitch base body according to the embodiment.
Fig. 15 shows a perspective view of the swing base body and a traction assembly according to the embodiment.
Figs. 16 to 18 schematically illustrate pivoting of the swing base body relative to the pitch base body according to the embodiment.
Fig. 19 shows a perspective side view of a swing base body according to another embodiment disclosed herein.
Fig. 20 shows a perspective end view of the swing base body according to the other embodiment.
Figs. 21 to 23 schematically illustrate pivoting of the swing base body relative to a pitch base body according to the other embodiment.
Fig. 24 shows a perspective view of a clamping instrument tail end according to yet another embodiment disclosed herein.
Fig. 25 shows a perspective cutaway view of the clamping instrument tail end according to the yet other embodiment.
Fig. 26 shows an exploded view of the clamping instrument tail end according to the yet other embodiment.
Fig. 27 shows a perspective view of a swing base body according to the yet other embodiment.
Fig. 28 shows a perspective view of a clamping instrument tail end according to still another embodiment.
Fig. 29 shows an exploded view of the clamping instrument tail end according to the still other embodiment.
Fig. 30 shows a perspective cutaway view of the clamping instrument tail end according to the still other embodiment.
Fig. 31 shows a perspective view of a swing base body and a traction assembly according to the still other embodiment.
Fig. 32 shows a perspective view of the swing base body according to the still other embodiment.
Fig. 33 shows an exploded view of the swing base body according to the still other embodiment.
Figs. 34 to 36 schematically illustrate pivoting of the swing base body relative to a pitch base body according to the still other embodiment.
Fig. 36a schematically illustrates arc-shaped wound sections and a straight intermediate section according to the still other embodiment.
Fig. 37 shows a perspective view of a clamping instrument tail end according to a fifth embodiment disclosed herein.
Fig. 38 shows a perspective view of a swing base body and a traction assembly according to the fifth embodiment.
Fig. 39 shows an exploded view of the clamping instrument tail end according to the fifth embodiment.
Fig. 40 shows a perspective view of the swing base body according to the fifth embodiment.
Fig. 41 shows an exploded view of a pitch base body according to the fifth embodiment.
Figs. 42 to 44 schematically illustrate pivoting of the swing base body relative to the pitch base body according to the fifth embodiment.
Fig. 45 shows a perspective view of a clamping instrument tail end according to a sixth embodiment disclosed herein.
Fig. 46 shows an exploded view of the clamping instrument tail end according to the sixth embodiment.
Fig. 47 shows a perspective view of a pitch base body according to the sixth embodiment.
Figs. 48 to 50 schematically illustrate pivoting of a swing base body relative to the pitch base body according to the sixth embodiment.
Fig. 51 is a schematic illustration of an energy instrument tail end according to a seventh embodiment disclosed herein.
Figs. 52 to 53 show a schematic diagram and a schematic exploded view of a curved component in the energy instrument tail end according to the seventh embodiment.
Fig. 54 and 55 show cutaway views of the energy instrument tail end according to the seventh embodiment (which has pivoted to the left in Fig. 55).
Figs. 56 to 57 schematically illustrate yaw and pitch base bodies according to the seventh embodiment.
Fig. 58 is a schematic illustration of a swing base body according to an eighth embodiment disclosed herein.
Figs. 59 to 60 show a schematic diagram and a schematic exploded view of an energy instrument tail end according to a ninth embodiment disclosed herein.
Fig. 61 is a schematic illustration of a swing base body according to the ninth embodiment.
Fig. 62 is a schematic illustration of a pitch base body according to the ninth embodiment.
Fig. 63 schematically illustrates an energy instrument tail end according to a tenth embodiment disclosed herein.
Fig. 64 shows a schematic exploded view of the energy instrument tail end according to the tenth embodiment.
Fig. 65 schematically illustrates an elastic adapter according to the tenth embodiment.
Fig. 66 schematically illustrates an adapter conductor according to the tenth embodiment.
Fig. 67 schematically illustrates an energy instrument tail end according to an eleventh embodiment disclosed herein.
Fig. 68 shows a schematic exploded view of the energy instrument tail end according to the eleventh embodiment.
Fig. 69 schematically illustrates an energy instrument tail end according to a twelfth embodiment disclosed herein.
Fig. 70 shows a schematic exploded view of the energy instrument tail end according to the twelfth embodiment.
Fig. 71 schematically illustrates a second energy delivery jaw according to the twelfth embodiment, which is being in a connected state.
Fig. 72 schematically illustrates first and second energy transmission cables according to the twelfth embodiment.

### DETAILED DESCRIPTION

The above objects, features and advantages of this application will become more apparent upon reading the following detailed description of specific embodiments disclosed herein in conjunction with the accompanying drawings. In the following description, numerous details are set forth in order to provide a thorough understanding of the application. However, this application may be practiced in many other forms than those described herein, and those skilled in the art can make similar improvements without deviating from the spirit of the application. Accordingly, the present application is not limited to the specific embodiments disclosed below.

It will be understood that terms such as "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. may be used herein to describe directional or positional relationships based on the orientations shown in the figures. They are intended merely to facilitate and simplify the explanation of the application and do not indicate or imply that the stated components or elements have to assume, or be constructed or operated in, particular orientations. Therefore, they are not to be construed as limiting the application.

In addition, as used herein, the terms "first", "second" and the like are intended only for illustration and are not to be construed as denoting or implying relative importance, or as implicitly indicating any particular number of the referenced items. Accordingly, defining an item with "first", "second" or the like is an explicit or implicit indication of the presence of at least one such item. As used herein, the term "plurality" means "at least two", such as two or three, unless otherwise clearly defined.

As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

As used herein, unless otherwise clearly specified or defined, when a first feature is described as being "above" or "below" a second feature, it may be either in direct contact with the second feature, or in indirect contact with one or more intervening media being present therebetween. When a first feature is referred to as being "on", "above" or "on top of" a second feature, it may be right or obliquely on, above or on top of the second feature, or simply located at a higher level than the second feature. When a first feature is referred to as being "under", "below" or "at bottom of" a second feature, it may be right or obliquely under, below or at bottom of the second feature, or simply located at a lower level than the second feature.

It should be noted that when a component is referred to as being "secured" to or "disposed" on another component, it may be directly on the other component, or intervening components may be present. When a component is referred to as being "connected" or "coupled" to another component, it can be directly connected or coupled to the other component, or intervening components may be present. As used herein, the terms "vertical", "horizontal", "upper", "lower", "left", "right" and the like are merely illustrative and do not represent the only embodiment possible.

To more clearly describe clamping instrument tail ends and surgical instruments proposed herein, the term "distal" is used herein to refer to an end farther away from an operator during surgical operation. For example, a "distal" end face of a pitch base body 1000 is an end face thereof located away from an operator. Moreover, the term "proximal" is used herein to refer to an end closer to an operator during surgical operation. For example, a "proximal" end face of a swing base body 2000 is an end face thereof closer to an operator. Unless defined otherwise, all technical and scientific terms used herein have the same meaning as commonly understood by one of ordinary skill in the art to which this application belongs. The terminology used herein is for the purpose of describing particular embodiments only and is not intended to be limiting of the application.

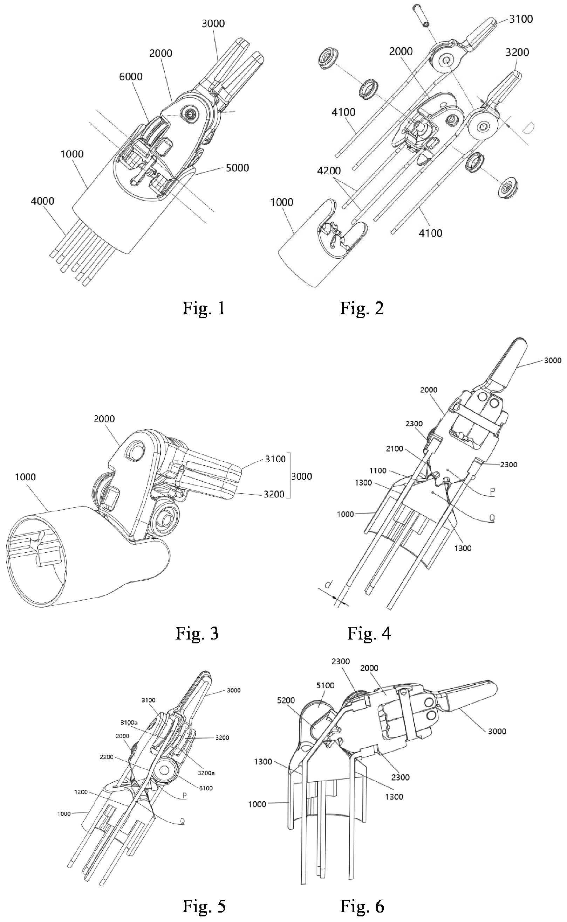

Referring to Figs. 1 to 18, in an embodiment disclosed herein, there is provided a clamping instrument tail end including a pitch base body 1000 and a swing base body 2000. In one embodiment, a terminal clamping body 3000 may be movably assembled on the swing base body 2000, and the terminal clamping body 3000 is controlled by a traction assembly 4000. The terminal clamping body 3000 may be implemented by one, two or more clamping elements. For example, it may consist of only a single clamping element, which may be implemented like an electrosurgical hook or spatula, or as a device with a central aspiration tube, such as an aspirator, used to flexibly suck away blood and other fluids generated during surgery. When the terminal clamping body 3000 has two or more clamping elements, these may be controlled to enable opening and closing of the terminal clamping body 3000. For example, the pitch base body 1000 may define a first traction channel 1200, and the swing base body 2000 may define a guide channel 2200. The traction assembly 4000 may include at least one first traction body 4100, each first traction body 4100 passes through a first traction channel 1200 and a guide channel 2200 and controllably coupled to the terminal clamping body 3000. In addition, an energy component for delivering energy may be movably assembled on the swing base body 2000, instead of a movably assembled terminal clamping body 3000. Non-limiting examples of the energy component may include an electrosurgical hook, an electrosurgical spatula and bipolar electrosurgical forceps. Each first traction body 4100 may be selected as a wire or belt.

The distal end face of the pitch base body 1000 is provided with a first contact boss 1100 so that the entire first contact boss 1100, except for its interface with the distal end face of the pitch base body 1000, is raised over, i.e., located above, the distal end face of the pitch base body 1000. In particular, it protrudes distally over the distal end face of the pitch base body 1000.

The pitch base body 1000 may define at least two first traction channels 1200, the first traction channels 1200 each distally terminate with a distal opening at the distal end face of the pitch base body 1000. This means that the distal opening of each first traction channel 1200 is located not above the distal end face of the pitch base body 1000. In particular, the distal opening may be located distally below the distal end face of the pitch base body 1000. First traction bodies 4100 passed through the first traction channels 1200 may deflect at their distal openings. Thus, such deflection is allowed to take place at the distal end face of the pitch base body 1000.

The proximal end face of the swing base body 2000 is provided with a second contact boss 2100 so that the entire second contact boss 2100, except for its interface with the proximal end face of the swing base body 2000 is raised over, i.e., located above, the proximal end face of the swing base body 2000. In particular, it protrudes proximally over the proximal end face of the swing base body 2000. The swing base body 2000 may define at least two guide channels 2200, the first traction channels 1200 each terminate with a proximal opening at the proximal end face of the swing base body 2000. First traction bodies 4100 passed through the guide channels 2200 may deflect at their proximal openings. Thus, such deflection is allowed to take place at the proximal end face of the swing base body 2000.

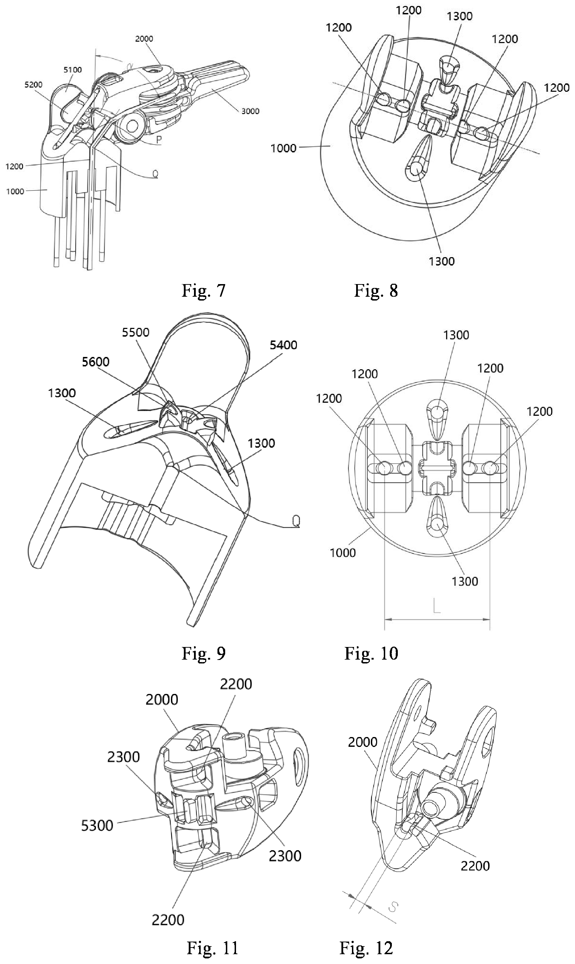

The swing base body 2000 is pivotably assembled with the pitch base body 1000, for example, using a linkage, multiple shafts or another mechanism. Pivoting of the swing base body 2000 relative to the pitch base body 1000 occurs with the first and second contact bosses 1100, 2100 being in rolling contact with each other. Referring to Fig. 7, as the swing base body 2000 is pivoting relative to the pitch base body 1000, the rolling contact of the first and second contact bosses 1100, 2100 occurs always distally above the distal end face of the pitch base body 1000 and proximally above the proximal end face of the swing base body 2000, concurrently with first traction bodies 4100 passed through the first traction channels 1200 deflecting at the distal end face of the pitch base body 1000 and first traction bodies 4100 passed through the guide channels 2200 deflecting at the proximal end face of the swing base body 2000.

Continued reference is made to Fig. 7. As can be seen from the figure, when the swing base body 2000 pivots by an angle α relative to the pitch base body 1000, each first traction body 4100 may deflect at two points by angles β, the deflection angle β is less than the angle of pivot α. The differences of the angles of deflection and pivot β, α depend on heights of the first and second contact bosses 1100, 2100, and this application is not limited to any particular values of the differences. For example, β ≈ α/2 may be satisfied, resulting in more favorable radii of curvature and reduced winding angles of the first traction body 4100 at the points of deflection, and hence less wear of the first traction body 4100 and an extended service life of an instrument in which the first traction body 4100 is employed.

The rolling contact of the first and second contact bosses 1100, 2100 may occur in a stable manner between their spherical, curved or similar surfaces. For example, in one embodiment, the surface of the first contact boss 1100 defines a first arcuate contact path, and the surface of the second contact boss 2100 defines a second arcuate contact path. Moreover, the swing base body 2000 and the pitch base body 1000 may be pivotably assembled together so that the first and second arcuate contact paths are maintained in tangential rolling contact with each other, ensuring that pivoting of the swing base body 2000 relative to the pitch base body 1000 always occurs in a stable and predictable manner.

Referring to Fig. 8, the distal openings of the first traction channels 1200 are all aligned on a straight line parallel to a pivoting direction of the swing base body, and the proximal openings of the guide channels 2200 are all aligned on another straight line parallel to the pivoting direction of the swing base body. Those skilled in the art may arrange the first traction channels 1200 and/or the guide channels 2200 in any different way, as needed, without departing from the scope of the present application. Referring to Fig. 4, when the first and second arcuate contact paths are brought into tangential rolling contact with each other, a pivoting direction of the first arcuate contact path passes through an imaginary point Q, and a pivoting direction of the second arcuate contact path passes through another imaginary point P. In one embodiment, the tangential rolling contact of the first and second contact bosses 1100, 2100 is dynamic contact, and a plane normal to a dynamic tangent line between the two members is referred to herein as a dynamic normal plane 1000a. In addition, the first traction channels 1200 may correspond to the respective guide channels 2200, and the distal and proximal openings of each such pair may be connected by a straight line referred to herein as a line segment of deflection 1000b.

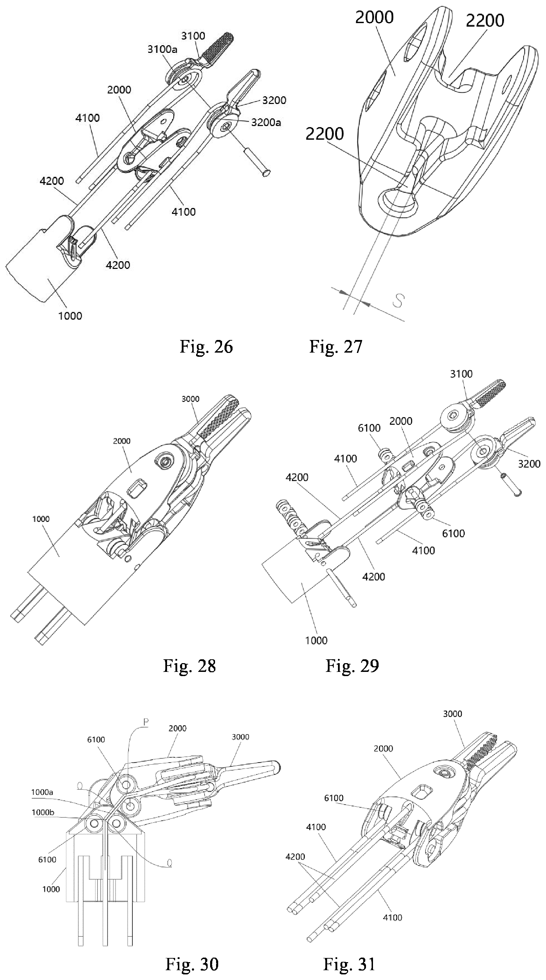

As labeled and shown in Fig. 30, the dynamic normal plane 1000a may be substantially parallel to the line segments of deflection 1000b, an angle between the dynamic normal plane 1000a and the line segments of deflection 1000b is maintained less than 10°, for example, 1°, 2°, 3°, 4°, 5°, 6°, 7°, 8°, 9° or 10°. This can minimize an unexpected torque exerted by the swing base body 2000 on the pitch base body 1000, which can adversely affect orientation accuracy of the pitch base body 1000. In one embodiment, denoting a diameter of each first traction body 4100 as d, it may also be ensured that any point on each line segment of deflection 1000b is spaced from the dynamic normal plane 1000a at a distance less than 4d. Before any pivoting of the swing base body 2000 occurs relative to the pitch base body 1000, they may be located in a plane referred to herein as an initial plane, and the dynamic normal plane 1000a may be oriented at an angle α with respect to the initial plane. The dynamic normal plane 1000a is oriented at an angle β with respect to the initial plane, pivoting may occur so that it is ensured that the angle β is greater than or equal to 0.5a - 15° and less than or equal to 0.5a + 15°.

In one embodiment, at least one of the pitch base body 1000 and the swing base body 2000 is provided with a motion-restraining mechanism 5000, the motion-restraining mechanism 5000 is used for maintaining the first arcuate contact path of the first contact boss 1100 and the second arcuate contact path of the second contact boss 2100 in tangential rolling contact. The motion-restraining mechanism 5000 may be implemented in various ways. For example, referring to Fig. 6, the motion-restraining mechanism 5000 may include a first lateral stop member 5100 and a second lateral stop member 5200, the first lateral stop member 5100 is disposed on the pitch base body 1000, the second lateral stop member 5200 is disposed on the swing base body 2000. The first and second lateral stop members 5100, 5200 may be brought into restraining contact with each other, the mutual restraining contact between the first lateral stop member 5100 and the second lateral stop member 5200 can be utilized to at least prevent the swing base body 2000 from any displacement.

The numbers and locations of first and second lateral stop members 5100, 5200 may be determined as required. For example, the first lateral stop member 5100 may be disposed on one side of the pitch base body 1000 and the second lateral stop member 5200 on a corresponding side of the swing base body 2000 to prevent the pitch base body 1000 and the swing base body 2000 from otherwise displacing towards the sides on which the first and second lateral stop members 5100, 5200 are disposed, during pivoting of the swing base body 2000 relative to the pitch base body 1000. Alternatively, two first lateral stop members 5100 may be provided on respective opposite sides of the pitch base body 1000 and two second lateral stop members 5200 of the swing base body 2000 to prevent the pitch base body 1000 and the swing base body 2000 from otherwise displacing toward either side on which the first and second lateral stop members 5100, 5200 are disposed, during pivoting of the swing base body 2000 relative to the pitch base body 1000. This can ensure that the first and second arcuate contact paths are strictly maintained in tangential rolling contact during pivoting of the swing base body 2000 relative to the pitch base body 1000.

Referring to Figs. 9 to 11, in one embodiment, the motion-restraining mechanism 5000 includes a restraining tooth 5300 and a restraining socket 5400, the restraining tooth 5300 and the restraining socket 5400 are separately provided on the pitch base body 1000 and the swing base body 2000. The restraining tooth 5300 can engage in the restraining socket 5400, thereby maintaining the first arcuate contact path of the first contact boss 1100 in tangential rolling contact with the second arcuate contact path of the second contact boss 2100. The restraining socket 5400 may be involute, cycloidal or the like, without limiting the scope of the application in any sense.

In one embodiment, the restraining socket 5400 is provided on the pitch base body 1000, and two lateral stop protrusions 5500 are also provided on the pitch base body 1000, the two lateral stop protrusions 5500 are provided on opposite sides of an opening of the restraining socket 5400. When the restraining tooth 5300 engages in the restraining socket 5400, the lateral stop protrusions 5500 result in heightening of an upper edge of the restraining socket 5400 on the opposite sides of its opening, which is equivalent to deepening of the restraining socket 5400, and are configured to come into contact with, and hence restrict, sides of the restraining tooth 5300, ensuring stable engagement of the restraining tooth 5300 in the restraining socket 5400. The restraining tooth 5300 may be pointed or rounded.

The pivotability of the swing base body 2000 relative to the pitch base body 1000 may be provided in various manners. For example, in one embodiment, the traction assembly 4000 includes at least two second traction bodies 4200, and the pitch base body 1000 defines at least two second traction channels 1300. Each of the second traction bodies 4200 is passed through a respective one of the second traction channels 1300 and connected to the swing base body 2000. In an example as shown in Fig. 4, the traction assembly 4000 includes two second traction bodies 4200, and the pitch base body 1000 defines two second traction channels 1300. Each of the second traction bodies 4200 is passed through a respective one of the second traction channels 1300 and connected to the swing base body 2000. Pulling any of the second traction bodies 4200 can cause the swing base body 2000 to correspondingly pivot relative to the pitch base body 1000. The lateral stop protrusions 5500 may define respective avoidance recesses 5600 for avoiding interference with the second traction bodies 4200. The second traction bodies 4200 may be selected as wires or belts.

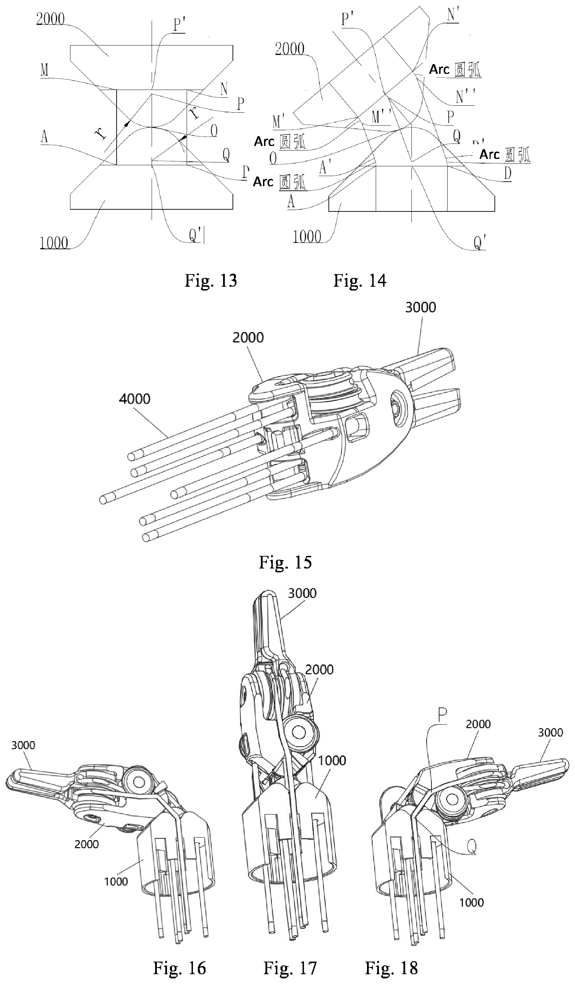

Referring to Figs. 12 and 13, the traction assembly 4000 includes two symmetrical second traction bodies 4200, which are fixed at one end to the swing base body at respective points M and N and passed at the other end through respective two second traction channels 1300 (labeled as A and D) in the pitch base body 1000. In an initial configuration, AM = DN = 2r. Assume lengths of sections of the two second traction bodies 4200 on opposite sides of the swing base body 2000 and the pitch base body 1000 becomes AM' and DN' as a result of pivoting of the swing base body 2000 relative to the pitch base body 1000. The three points A, Q and D are collinear, and Q is the midpoint of AD. Likewise, the three points M, P and N are collinear, and P is the midpoint of MN. Therefore, ADN'M' is an isosceles trapezoid, and AM' + DN' = 2PQ = 2r = AM + DN, i.e., AM' - AM = DN - DN', is satisfied, meaning that the sections of the left and right second traction bodies 4200 have experienced equal length changes. In one embodiment, the second traction channels 1300 may have filleted edges at their openings. In this case, the two symmetrical second traction bodies 4200 may also be fixed at one end to the swing base body at respective points M and N and passed at the other end through the respective two second traction channels 1300 (A and D) in the pitch base body 1000. In an initial configuration, AM = DN = 2P'Q'. Assume lengths of sections of the two second traction bodies 4200 on oppsite sides of the swing base body 2000 and the pitch base body 1000 becomes AA', 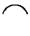 + A'M" + M'M", 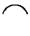 and DD', 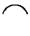 + D'N" + N'N", 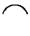 as a result of the yaw base 2000 pivoting relative to the pitch base 1000. Although the three points A, Q and D are not collinear, AQ ≈ QD. Likewise. although the three points M. P and N are not collinear PM ≈ PN. Therefore, AA', 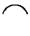 + A'M" + M'M", 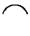 + DD', 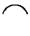 + D'N" + N'N", 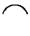 ≈2P'Q' = AM + DN, i.e., AA', 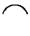 + A'M" + M'M", 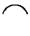 - AM ≈ DN" + N'N", 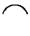 - DN', is satisfied, meaning that the sections of the left and right second traction bodies 4200 have experienced approximately equal length changes.

In one embodiment, each second traction channel 1300 has a distal opening at the distal end face of the pitch base body 1000, and all distal openings of the second traction channel 1300 are aligned on a straight line perpendicular to the pivoting direction of the swing base body. The swing base body 2000 may be provided with at least two traction connecting features 2300, each traction connecting feature 2300 corresponds to a respective one of the second traction channels 1300, and each of the second traction bodies 4200 may be connected to a respective one of the traction connecting features 2300 on the swing base body 2000. Non-limiting examples of the traction connecting features 2300 may include through-holes, hooks and fasteners.

The terminal clamping body 3000 may be any of various commonly known types. For example, in one embodiment, the terminal clamping body 3000 includes a first clamping jaw 3100 and a second clamping jaw 3200, each of the first clamping jaw 3100 and the second clamping jaw 3200 is able to pivot relative to the swing base body 2000 about a fixed axis of pivot. Alternatively, the terminal clamping body 3000 may include more than two clamping jaws. In any case, the clamping jaws may be configured to move in an interdependent manner. However, the present application is not so limited.

Continued reference is made to Fig. 8. In the illustrated example, the pitch base body 1000 defines four first traction channels 1200, and the swing base body 2000 defines two guide channels 2200. Each pair of first traction channels 1200 corresponds to a respective one of the guide channels 2200. The traction assembly 4000 includes two first traction bodies 4100, which are wound respectively on a first clamping jaw 3100 and a second clamping jaw 3200 and configured to cause the first clamping jaw 3100 or the second clamping jaw 3200 to pivot about respective fixed axes of pivot. Opposite end sections of each first traction body 4100 are threaded through a respective pair of first traction channels 1200. That is, the four end portions of the two first traction bodies 4100 are threaded through the respective four first traction channels 1200. Each first traction body 4100 may be integral, wherein the opposite end sections refer to its two sections extending both from a point, where the traction is fixed to the first or second clamping jaw 3100, 3200, to its respective opposite ends. Alternatively, each first traction body 4100 may be composed of two separate component tendons, which are both tied at one end to the first or second clamping jaw 3100, 3200 and thus form the first traction body 4100 connected to the first or second clamping jaw 3100, 3200.

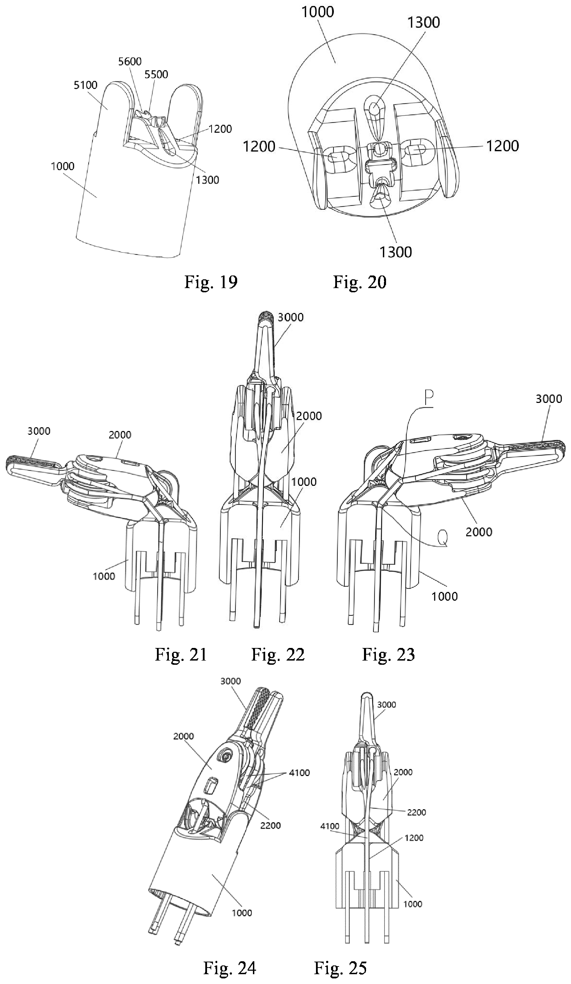

Referring to Figs. 19 to 23, in one embodiment, the pitch base body 1000 defines two first traction channels 1200, and the swing base body 2000 defines two guide channels 2200. Each of the first traction channels 1200 corresponds to a respective one of the guide channels 2200. The traction assembly 4000 includes two first traction bodies 4100, which are wound respectively on a first clamping jaw 3100 and a second clamping jaw 3200 and configured to cause the first clamping jaw 3100 or the second clamping jaw 3200 to pivot about respective fixed axes of pivot. Each first traction body 4100 is dynamically divided at a point, where the winding occurs, into two opposite end sections threaded through the respective first traction channels 1200. Thus, there are two end sections in each first traction channel 1200.

At least one of the pitch base body 1000 and the swing base body 2000 is provided with a guide mechanism 6000. The guide mechanism 6000 may be configured to guide two first traction bodies 4100 so as to allow them to be correctly wound respectively on the first clamping jaw 3100 and the second clamping jaw 3200. In one embodiment, the first clamping jaw 3100 has first annular winding grooves 3100a configured for winding engagement of the first traction bodies 4100 therein, and the second clamping jaw 3200 has second annular winding grooves 3200a configured for winding engagement of second traction bodies 4200 therein. The first and second annular winding grooves 3100a, 3200a each define a winding diameter D, and the first traction bodies 4100 have a diameter d.

Fig. 10 shows distal openings of two first traction channels 1200 that opposite end sections of a first traction body 4100 are passed through being spaced from each other at a straight-line distance L. When there are four first traction channels 1200 and two first traction bodies 4100, opposite end sections of each first traction body 4100 may be threaded through a symmetrical pair of first traction channels 1200. In this case, the straight-line distance L may be measured either between distal openings of the two outermost symmetrical first traction channels 1200, or between distal openings of the two innermost first traction channels 1200, and may satisfy D - 5d < L < D + 5d.

The guide mechanism 6000 may take various forms, as shown in Figs. 24 to 27. In Fig. 27, each traction channel has a minimum width s, and each first traction body 4100 has a diameter d, where d < s < d + 1 mm. The guide mechanism 6000 may include a lubricating coating on inner walls of the guide channels 2200. This lubricating coating may be obtained by a coating process, and may be implemented as a nylon plating, a PTFE plating, a Parylene coating, etc. Additionally, each of the swing base body 2000 and the pitch base body 1000 may be made of a material with a low coefficient of friction. These can reduce wear of a first traction body 4100 and extend its service life, eventually extending the service life of an instrument in which the first traction body 4100 is employed. Further, the use of the lubricating coating can reduce the number of components, resulting in cost reductions and easier fabrication.

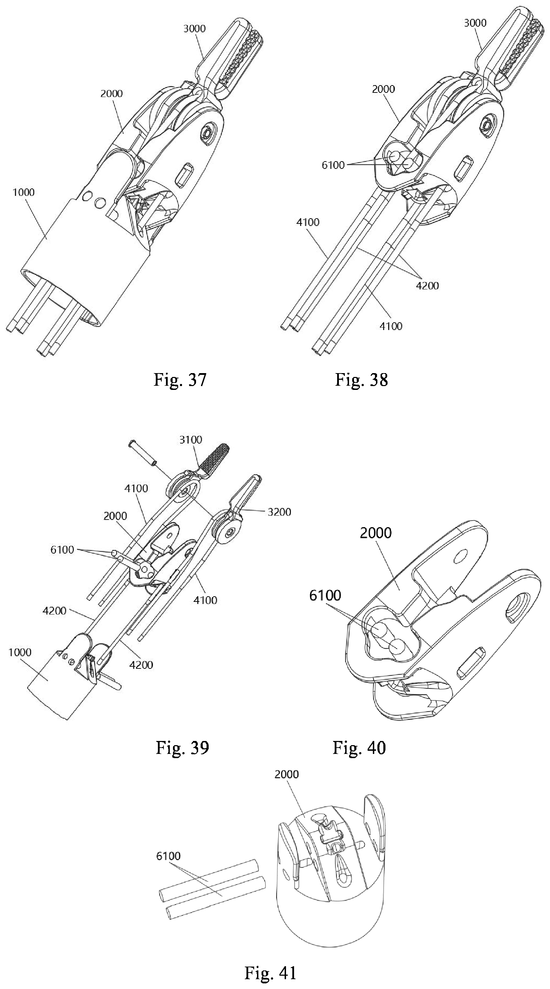

Referring to Figs. 28 to 44, the guide mechanism 6000 may also include multiple rolling guide elements 6100 disposed on at least one side of at least one of the first traction channels 1200 and the guide channels 2200. First traction bodies 4100 may be brought into rolling contact with, and thus guided by, the rolling guide elements 6100, and rolling friction between them enables more efficient power transmission. Referring to Figs. 28 to 36, the rolling guide elements 6100 may be implemented as guide pulleys, and the number of guide pulleys may depend on the number of first traction bodies 4100 used and may suffice to provide desirable guidance to the first traction bodies 4100. Referring to Figs. 28 to 36, the rolling guide elements 6100 may be alternatively implemented as guide posts. Likewise, the number of guide posts may depend on the number of first traction bodies 4100 used and may suffice to provide desirable guidance to the first traction bodies 4100. However, the present application is not so limited.

With continued reference to Figs. 28 to 36, an even number of rolling guide elements 6100 may be included and paired, and the rolling guide elements 6100 of each pair may be arranged in symmetry on respective opposite sides of at least one of the first traction channels and the guide channels. For example, in an example as shown in Fig. 30, on opposite sides of each of the first traction channels and the guide channels, two rolling guide elements 6100 are arranged in symmetry. That is, the first traction channels and the guide channels are provided with a total of four rolling guide elements 6100. First traction bodies 4100 may be guided and deflected by these two pairs of rolling guide elements 6100 in a stably movable manner. As a result, the service life of the first traction bodies 4100 can be extended.

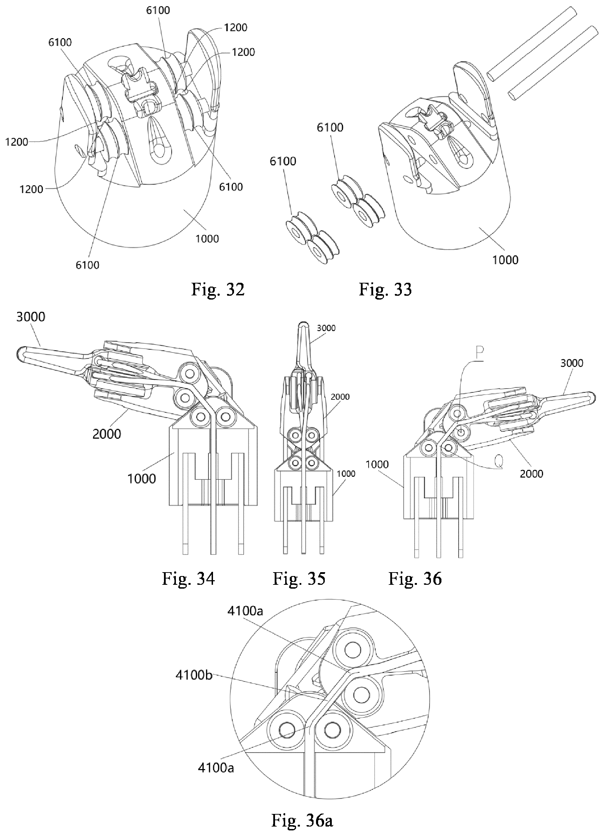

Eight rolling guide elements 6100 may be provided in the form of rolling wheels each defining two symmetrical rolling grooves on its circumference. Four of the rolling wheels may be symmetrically assembled on the distal end face of the pitch base body 1000. Referring to Figs. 34 to 36, here, by "symmetrical assembled", it is intended to mean that the rolling wheels are symmetrically arranged not only along the pivoting direction of the swing base body 2000 but also in a direction perpendicular to the pivoting direction of the swing base body 2000. Such symmetry in both the lateral and longitudinal directions ensures accurate guidance for first traction bodies 4100. The other four rolling wheels may be symmetrically assembled on the proximal end face of the swing base body 2000. Similarly, here, by "symmetrical assembled", it is intended to mean that the rolling wheels are symmetrically arranged not only along the pivoting direction of the swing base body 2000 but also in the direction perpendicular to the pivoting direction of the swing base body 2000. Such symmetry in both the lateral and longitudinal directions ensures accurate guidance for first traction bodies 4100. Multiple first traction bodies 4100 can be slidably engaged in sets of corresponding rolling grooves on the rolling wheels so as to be able to be guided to move in a desired direction. With this arrangement, higher overall drive stability can be achieved.

Referring to Fig. 36a, when a first traction body 4100 engages into a rolling groove on the circumference of a rolling wheel, it will have an arc-shaped wound section 4100a. If a first traction body 4100 engages into rolling grooves on the circumference of two rolling wheels arranged respectively on the pitch base body 1000 and the swing base body 2000, it will have two arc-shaped wound sections 4100a spaced apart and joined by a straight intermediate section 4100b. While the swing base body 2000 is pivoting, the straight intermediate section 4100b of each first traction body 4100 between the two arc-shaped wound sections 4100a may experience a total length change not exceeding 5 mm. Thus, with the rolling guide elements 6100, much more stable and more accurate pivoting can be achieved.

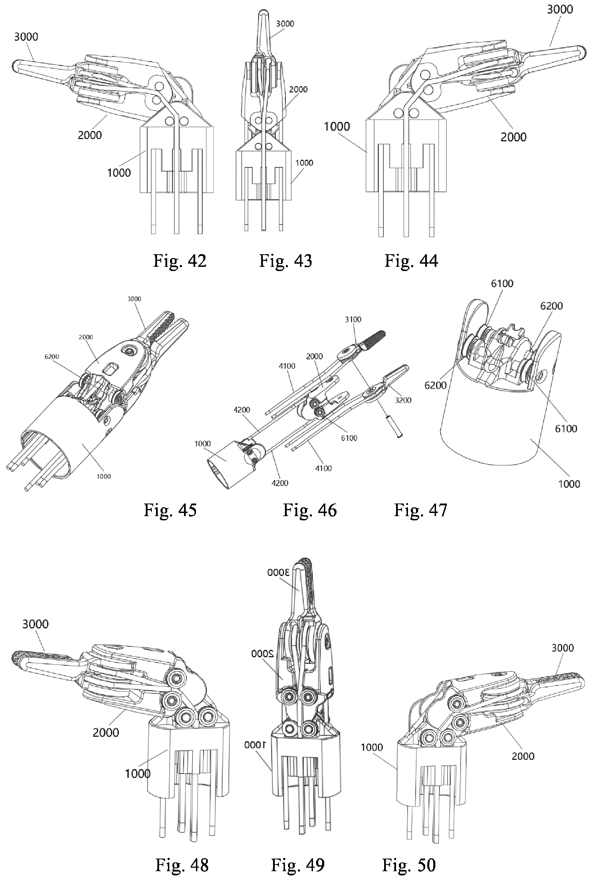

Additionally, referring to Figs. 45 to 50, the guide mechanism 6000 may include multiple sliding guide elements 6200, the multiple sliding guide elements 6200 are disposed on at least one side of at least one of the first traction channels 1200 and the wire guide channels 2200. First traction bodies 4100 may be brought into sliding contact with, and thus guided by, the sliding guide elements 6200. Such contact may occur between either some or all of the first traction bodies 4100 and the sliding guide elements 6200. An even number of sliding guide elements 6200 may be included and paired, and the sliding guide elements 6200 of each pair may be arranged in symmetry on respective opposite sides of at least one of the first traction channels and the guide channels. Those skilled in the art may select an appropriate number of sliding guide elements 6200 and arrange them appropriately, without departing from the scope of this application.

The guide mechanism may include both multiple rolling guide elements 6100 and multiple sliding guide elements 6200. That is, multiple rolling guide elements 6100 may be used in combination with multiple sliding guide elements 6200. For example, the total sum of the number of rolling guide elements 6100 and the number of sliding guide elements 6200 may be an even number. These rolling and sliding guide elements 6100, 6200 may be paired, and the guide elements in each pair may be arranged in symmetry on respective opposite sides of at least one of the first traction channels and the guide channels. For example, in one embodiment, with continued reference to Figs. 45 to 50, each first traction body 4100 may have two stressed wire sections, namely, a first stressed wire section and a second stressed wire section. The first and second stressed wire sections are stressed by different forces. Specifically, the first stressed wire section is stressed by a force that is greater than a force exerted on the second stressed wire section. The first stressed wire section may be subject to a larger average force, which may vary, for example, in the range of 15 N to 80 N. The second stressed wire section may be subject to a smaller force, which may vary, for example, in the range of 0 N to 15 N. The first stressed wire section is in rolling contact with and thereby guided by a rolling guide element 6100, while the second stressed wire section is in sliding contact with and thereby guided by a sliding guide element 6200. Since the first stressed wire sections are stressed to drive opening or closing of the terminal clamping body 3000, guiding them by rolling contact can provide sufficient drive power and resulting in an extended life span. On the contrary, the second stressed wire sections do not contribute to driving opening or closing of the terminal clamping body 3000, guiding them by lower-cost sliding contact can ensure proper functionality while minimizing cost.

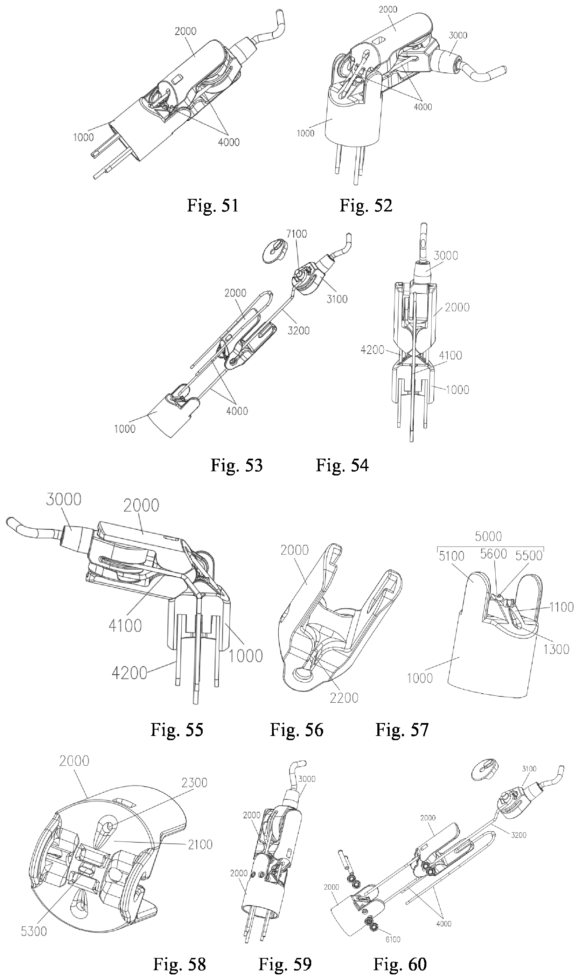

Referring to Figs. 51 to 57, an energy instrument tail end according to an embodiment disclosed herein includes a pitch base body 1000, a swing base body 2000 and an energy component 3000. The pitch base body 1000 is provided with a first contact boss 1100 protruding its distal end face and defines first traction channels 1200 each having a distal opening located at the distal end face of the pitch base body 1000. The swing base body 2000 is provided with a second contact boss 2100 protruding from its proximal end face and is assembled with the pitch base body 1000 so as to reciprocally pivotable relative to the pitch base body 1000 in a predefined direction. For example, it may pivot forward or backward in the predefined direction, with the first and second contact bosses 1100, 2100 remaining in rolling contact with each other. The swing base body 2000 defines transition guide channels 2200 each having a proximal opening located at the proximal end face of the swing base body 2000. The energy component 3000 includes an energy release body 3100 and an energy transmission cable 3200. The energy release body 3100 is assembled on the swing base body, and the energy transmission cable 3200 is passed through a first traction channel 1200 and a transition guide channel 2200 and connected to the energy release body 3100. Non-limiting examples of the energy release body 3100 may include an electrosurgical hook, an electrosurgical spatula, bipolar electrosurgical forceps, etc.

The first contact boss 1100 is provided on the distal end face of the pitch base body 1000 so that the first contact boss 1100's entirety, except for its interface with the distal end face of the pitch base body 1000, is raised over, i.e., located above, the distal end face of the pitch base body 1000. In particular, it protrudes distally over the distal end face of the pitch base body 1000.

The distal openings of the first traction channels 1200 in the pitch base body 1000 are all located at the distal end face of the pitch base body 1000. This means that the distal openings of the first traction channels 1200 are all located not above the distal end face of the pitch base body 1000. In particular, the distal openings may be located distally below the distal end face of the pitch base body 1000. The energy transmission cable 3200 passed through the first traction channel 1200 may deflect at its distal opening. Thus, the deflection is allowed to take place at the distal end face of the pitch base body 1000.

The second contact boss 2100 is provided on the proximal end face of the swing base body 2000 so that the second contact boss 2100's entirety, except for its interface with the proximal end face of the swing base body 2000, is raised over, i.e., located above, the proximal end face of the swing base body 2000. In particular, it protrudes proximally over the proximal end face of the swing base body 2000. The proximal openings of the transition guide channels 2200 in the swing base body 2000 are all located at the proximal end face of the swing base body 2000. The energy transmission cable 3200 passed through the transition guide channel 2200 may deflect at its proximal opening. Thus, the deflection is allowed to take place at the proximal end face of the swing base body 2000.

The swing base body 2000 is pivotably assembled with the pitch base body 1000, for example, using a linkage, multiple shafts or another mechanism. Pivoting of the swing base body 2000 relative to the pitch base body 1000 occurs with the first and second contact bosses 1100, 2100 being in rolling contact with each other. As the swing base body 2000 is pivoting relative to the pitch base body 1000, the rolling contact of the first and second contact bosses 1100, 2100 occurs always distally above the distal end face of the pitch base body 1000 and proximally above the proximal end face of the swing base body 2000, concurrently with the energy transmission cable 3200 passed through the first traction channel 1200 deflecting at the distal end face of the pitch base body 1000 and the energy transmission cable 3200 passed through the transition guide channel 2200 deflecting at the proximal end face of the swing base body 2000.

Assume that when the swing base body 2000 pivots by an angle α relative to the pitch base body 1000, the energy transmission cable 3200 deflects at two points by angles β. The angles of deflection β are less than the angle of rotation α, and the differences of the angles of deflection and pivot β, α depend on heights of the first and second contact bosses 1100, 2100. This application is not limited to any particular values of the differences. For example, β ≈ α/2 may be satisfied, resulting in more favorable radii of curvature and reduced winding angles of the energy transmission cable 3200 at the points of deflection, and hence less wear of the energy transmission cable 3200 and an extended service life of an instrument in which the energy transmission cable 3200 is employed.

The rolling contact of the first and second contact bosses 1100, 2100 may occur in a stable manner between their spherical, curved or similar surfaces. For example, in one embodiment, the surface of the first contact boss 1100 defines a first arcuate contact path, and the surface of the second contact boss 2100 defines a second arcuate contact path. Moreover, the swing base body 2000 and the pitch base body 1000 may be pivotably assembled together so that the first and second arcuate contact paths are maintained in tangential rolling contact with each other, ensuring that pivoting of the swing base body 2000 relative to the pitch base body 1000 always occurs in a stable and predictable manner.

In one embodiment, the energy instrument tail end includes a traction assembly 4000, the traction assembly 4000 includes a first traction body 4100, which may be selected as a wire or belt. The number of first traction channels 1200 and the number of transition guide channels 2200 may be both at least two. For example, there are two first traction channels 1200 and two transition guide channels 2200, with the energy transmission cable 3200 being threaded through one of the first traction channels 1200 and one of the transition guide channels 2200. The energy release body 3100 is pivotably coupled to the swing base body, with opposite end sections of the first traction body 4100 being passed through respective two of the first traction channels 1200 and respective two of the transition guide channels 2200 and configured for winding engagement with the energy release body 3100. Pulling the opposite ends of the first traction body 4100 can cause the energy release body 3100 to reciprocally pivot in a predefined direction relative to the swing base body. For example, it may be pivoted forward or backward in the predefined direction.

The first contact boss 1100 is arranged on the pitch base body 1000 so as to protrude over the distal end face thereof, and the first traction body 4100 threaded through the first traction channels 1200 may deflect at the distal openings of the first traction channels 1200. Thus, the deflection is allowed to take place at the distal end face of the pitch base body 1000. The second contact boss 2100 is arranged on the swing base body 2000 so as to protrude over the proximal end thereof, and the first traction body 4100 threaded through the transition guide channels 2200 may deflect at the proximal openings of the transition guide channels 2200. Thus, the deflection is allowed to take place at the proximal end face of the swing base body 2000.

As with the energy transmission cable 3200, pivoting of the swing base body 2000 relative to the pitch base body 1000 occurs with the first and second contact bosses 1100, 2100 remaining in rolling contact with each other distally above the distal end face of the pitch base body 1000 and proximally above the proximal end face of the swing base body 2000 and with the first traction body 4100 passed through the first traction channels 1200 deflecting at the distal end face of the pitch base body 1000 and the first traction body 4100 passed through the transition guide channels 2200 deflecting at the proximal end face of the swing base body 2000.

Assume that when the swing base body 2000 pivots by an angle α relative to the pitch base body 1000, each section of the first traction body 4100 deflects at two points by angles β. The angles of deflection β are less than the angle of rotation α, and the differences of the angles of deflection and pivot β, α depend on the heights of the first and second contact bosses 1100, 2100. This application is not limited to any particular values of the differences. For example, β ≈ α/2 may be satisfied, resulting in more favorable radii of curvature and reduced winding angles of the first traction body 4100 at the points of deflection, and hence less wear of the first traction body 4100 and an extended service life of an instrument in which the first traction body 4100 is employed.

In one embodiment, at least one of the pitch base body 1000 and the swing base body 2000 is provided with a motion-restraining mechanism 5000, the motion-restraining mechanism 5000 is used for maintaining the first contact boss 1100 in tangential rolling contact with the second contact boss 2100. The swing base body 2000 may include a first base portion 2400 and a second base portion 2500, both the first base portion 2400 and the second base portion 2500 are pivotably coupled to the pitch base body 1000. The motion-restraining mechanism 5000 may be provided on the first base portion 2400, with the second contact boss 2100 being arranged on the second base portion 2500. Those skilled in the art may arrange them appropriately as needed, without departing from the scope of the present application. The motion-restraining mechanism 5000 may take various forms. For example, as shown, the motion-restraining mechanism 5000 may include a first lateral stop member 5100 and a second lateral stop member 5200, the first lateral stop member 5100 is disposed on the pitch base body 1000, the second lateral stop member 5200 is disposed on the swing base body 2000. The first and second lateral stop members 5100, 5200 may be brought into restraining contact with each other, the mutual restraining contact between the first lateral stop member 5100 and the second lateral stop member 5200 can be utilized to at least prevent the swing base body 2000 from any displacement.

The numbers and locations of first and second lateral stop members 5100, 5200 may be determined as required. For example, the first lateral stop member 5100 may be disposed on one side of the pitch base body 1000 and the second lateral stop member 5200 on a corresponding side of the swing base body 2000 to prevent the pitch base body 1000 and the swing base body 2000 from otherwise displacing towards the sides on which the first and second lateral stop members 5100, 5200 are disposed, during pivoting of the swing base body 2000 relative to the pitch base body 1000. Alternatively, two first lateral stop members 5100 may be provided on respective opposite sides of the pitch base body 1000 and two second lateral stop members 5200 of the swing base body 2000 to prevent the pitch base body 1000 and the swing base body 2000 from otherwise displacing toward either side on which the first and second lateral stop members 5100, 5200 are disposed, during pivoting of the swing base body 2000 relative to the pitch base body 1000. This can ensure that the first and second arcuate contact paths are strictly maintained in tangential rolling contact during pivoting of the swing base body 2000 relative to the pitch base body 1000.

Referring to Figs. 56 to 58, in one embodiment, the motion-restraining mechanism 5000 includes a restraining tooth 5300 and a restraining socket 5400, which are separately provided on the pitch base body 1000 and the swing base body 2000. The restraining tooth 5300 can engage in the restraining socket 5400, thereby maintaining the first arcuate contact path of the first contact boss 1100 in tangential rolling contact with the second arcuate contact path of the second contact boss 2100. The restraining socket 5400 may be involute, cycloidal or the like, without limiting the scope of the application in any sense.

In one embodiment, the restraining socket 5400 is provided on the pitch base body 1000, and two lateral stop protrusions 5500 are also provided on the pitch base body 1000 on opposite sides of an opening of the restraining socket 5400. When the restraining tooth 5300 engages in the restraining socket 5400, the lateral stop protrusions 5500 result in heightening of an upper edge of the restraining socket 5400 on the opposite sides of its opening, which is equivalent to deepening of the restraining socket 5400, and are configured to come into contact with, and hence restrict, sides of the restraining tooth 5300, ensuring stable engagement of the restraining tooth 5300 in the restraining socket 5400. The restraining tooth 5300 may be pointed or rounded.

In one embodiment, the traction assembly 4000 includes at least two second traction bodies 4200, the first traction body 4100 is optionally selected as a wire or belt. Additionally, the pitch base body 1000 defines at least two second traction channels 1300. For example, the pitch base body 1000 may define two second traction channels 1300. Each of the second traction bodies 4200 is passed through a respective one of the second traction channels 1300 and connected to the swing base body 2000, in order to drive the swing base body 2000 to pivot relative to the pitch base body 1000. In the case of two second traction bodies 4200, they may be arranged in symmetry, fixed at one end to respective two symmetrical points on the swing base body, and passed at the other end through respective two second traction channels 1300 in the pitch base body 1000. The lateral stop protrusions 5500 may define respective avoidance recesses 5600 for avoiding interference with the second traction bodies 4200.

In one embodiment, each second traction channel 1300 has a distal opening at the distal end face of the pitch base body 1000, and all these distal openings are aligned on a straight line perpendicular to the pivoting direction of the swing base body. The swing base body 2000 may be provided with at least two traction connecting features 2300 each corresponding to a respective one of the second traction channels 1300, and each of the second traction bodies 4200 may be connected to a respective one of the traction connecting features 2300 on the swing base body 2000. Non-limiting examples of the traction connecting features 2300 may include through-holes, hooks and fasteners.

The pitch base body 1000 may define two first traction channels 1200, and the swing base body 2000 may define two guide channels 2200. Each of the first traction channels 1200 may correspond to a respective one of the guide channels 2200. The first traction body 4100 may be wound on the energy release body 3100 and configured to cause the energy release body 3100 to pivot about a fixed axis of pivot. Opposite end sections of the first traction body 4100 may be threaded through the respective first traction channels 1200 and the respective transition guide channels 2200, with the energy transmission cable 3200 being passed through one of the first traction channels 1200 and one of the transition guide channels 2200. Thus, the energy transmission cable 3200 is passed through the same first traction channel 1200 as one end section of the first traction body 4100 and through the same transition guide channel 2200 as one end section of the first traction body 4100.

The pitch base body 1000 may define three first traction channels 1200, and the swing base body 2000 may define three transition guide channels 2200. Each of the first traction channels 1200 may correspond to a respective one of the guide channels 2200. The first traction body 4100 may be wound on the energy release body 3100 and configured to cause the energy release body 3100 to pivot about a fixed axis of pivot. Opposite end sections of the first traction body 4100 may be threaded through respective two of the first traction channels 1200 and respective two of the transition guide channels 2200, with the energy transmission cable 3200 being passed through the remaining one of the first traction channels 1200 and the remaining one of the transition guide channels 2200. With this arrangement, the first traction channel 1200, through which the energy transmission cable 3200 is threaded, is different from the first traction channels 1200, through which the respective opposite end sections of the first traction body 4100 are threaded, and the transition guide channel 2200, through which the energy transmission cable 3200 is threaded, is different from the transition guide channels 2200, through which the respective opposite end sections of the first traction body 4100 are threaded. This can reduce possible wear resulting from rubbing of the energy transmission cable 3200 against the first traction body 4100. In one embodiment, the first traction channel 1200, through which the energy transmission cable 3200 is threaded, may have a curved transition surface 1400, which can reduce rubbing and abrading of the energy transmission cable 3200. A cable separation component 1500 may be disposed between the first traction channel 1200, through which the energy transmission cable 3200 is threaded, and the two first traction channels 1200, through which the first traction body 4100 is threaded, to separate the first traction body 4100 from the energy transmission cable 3200, additionally preventing mutual interference of the first traction body 4100 and the energy transmission cable 3200.

In one embodiment, at least one of the pitch base body 1000 and the swing base body 2000 is provided with a movement guide mechanism 6000 implemented as a lubricating coating, rolling guide elements 6100, sliding guide elements, or the like. The lubricating coating may be obtained by a coating process, and may be implemented as a nylon plating, a PTFE plating, a Parylene coating, etc. Additionally, each of the swing base body 2000 and the pitch base body 1000 may be made of a material with a low coefficient of friction. These can reduce wear of the first traction body 4100 and the energy transmission cable 3200 and extend their life span, eventually extending the service life of an instrument in which they are employed. Further, the use of the lubricating coating can reduce the number of components, resulting in cost reductions and easier fabrication.

In one embodiment, the movement guide mechanism 6000 includes multiple rolling guide elements 6100, the multiple rolling guide elements 6100 are disposed on at least one side of at least one of the first traction channels 1200 and transition guide channels. First traction bodies 4100 may be brought into rolling contact with the rolling guide elements 6100, thus the first traction bodies 4100 are guided thereby for winding onto the energy release body 3100. Rolling friction between them enables more efficient power transmission. Referring to Figs. 59 to 62, the rolling guide elements 6100 may be implemented as guide pulleys, and the number of guide pulleys may depend on the number of first traction bodies 4100 used and may suffice to provide desirable guidance to the first traction bodies 4100. The rolling guide elements 6100 may be alternatively implemented as guide posts. Likewise, the number of guide posts may depend on the number of first traction bodies 4100 used and may suffice to provide desirable guidance to the first traction bodies 4100. However, the present application is not so limited.

An even number of rolling guide elements 6100 may be included and paired, and the rolling guide elements 6100 of each pair may be arranged in symmetry on respective opposite sides of at least one of the first traction channels 1200 and the transition guide channels 2200. For example, on opposite sides of each of the first traction channels 1200 and the transition guide channels 2200, two rolling guide elements 6100 are arranged in symmetry. That is, the first traction channels 1200 and the transition guide channels 2200 are provided with a total of four rolling guide elements 6100. First traction bodies 4100 may be guided and deflected by these two pairs of rolling guide elements 6100 in a stably movable manner. As a result, the service life of the first traction bodies 4100 can be extended.

Eight rolling guide elements 6100 may be provided in the form of rolling wheels each defining two symmetrical rolling grooves on its circumference. Four of the rolling wheels may be symmetrically assembled on the distal end face of the pitch base body 1000. Here, by "symmetrical assembled", it is intended to mean that the rolling wheels are symmetrically arranged not only along the pivoting direction of the swing base body 2000 but also in a direction perpendicular to the pivoting direction of the swing base body 2000. Such symmetry in both the lateral and longitudinal directions ensures accurate guidance for first traction bodies 4100. The other four rolling wheels may be symmetrically assembled on the proximal end face of the swing base body 2000. Similarly, here, by "symmetrical assembled", it is intended to mean that the rolling wheels are symmetrically arranged not only along the pivoting direction of the swing base body 2000 but also in the direction perpendicular to the pivoting direction of the swing base body 2000. Such symmetry in both the lateral and longitudinal directions ensures accurate guidance for first traction bodies 4100. Multiple first traction bodies 4100 can be slidably engaged in sets of corresponding rolling grooves on the rolling wheels so as to be able to be guided to move in a desired direction. With this arrangement, higher overall drive stability can be achieved.

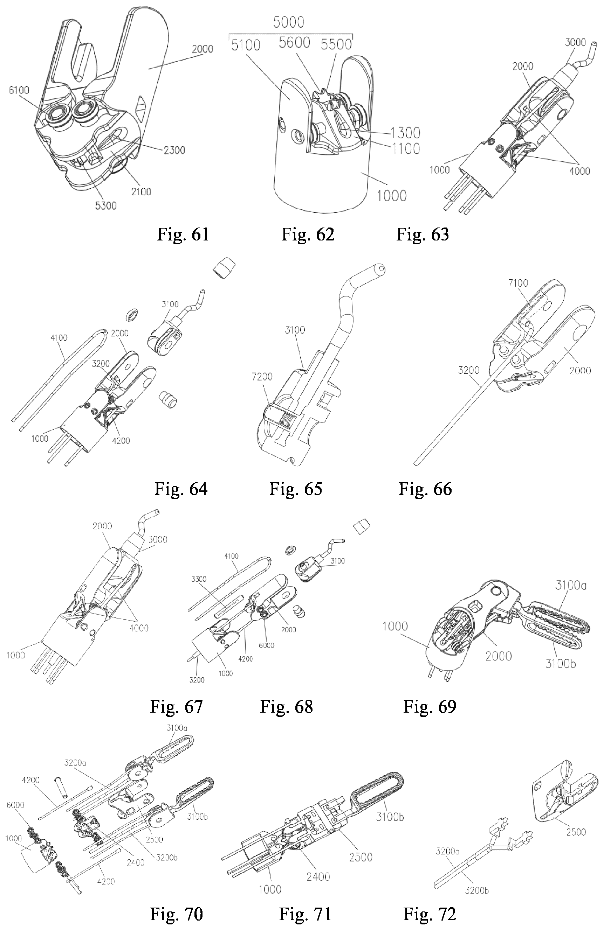

Assume that when the swing base body 2000 pivots by an angle α relative to the pitch base body 1000, the energy transmission cable 3200 deflects at two points by angles β. The angles of deflection β are less than the angle of rotation α, and the differences of the angles of deflection and pivot β, α depend on the heights of the first and second contact bosses 1100, 2100. This application is not limited to any particular values of the differences. For example, β ≈ α/2 may be satisfied, resulting in more favorable radii of curvature and reduced winding angles of the energy transmission cable 3200 at the points of deflection, and hence less wear of the energy transmission cable 3200 and an extended service life of an instrument in which the energy transmission cable 3200 is employed. The energy transmission cable 3200 may deflect around sliding guide elements 6200 on the pitch base body 1000 and the swing base body 2000 (or the first base portion 2400 of the swing base body 2000), which may be implemented as arc-shaped restraining means. Referring to Fig. 66, if needed, the sliding guide elements 6200 may be replaced with rolling guide elements 6100. This can improve reliability of the energy transmission cable 3200 and extend its life span.

When a first traction body 4100 engages into a rolling groove on the circumference of a rolling wheel, it will have an arc-shaped wound. If a first traction body 4100 engages into rolling grooves on the circumference of two rolling wheels arranged respectively on the pitch base body 1000 and the swing base body 2000, it will have two arc-shaped wound sections spaced apart and joined by a straight intermediate section While the swing base body 2000 is pivoting, the straight intermediate section of each first traction body 4100 between the two arc-shaped wound sections may experience a total length change not exceeding 5 mm. Thus, with the rolling guide elements 6100, much more stable and more accurate pivoting can be achieved. Such a consistent total length change can also be achieved for the energy transmission cable 3200 in a similar way, and further description thereof is omitted herein for the sake of brevity.

In one embodiment, the energy release body 3100 has annular winding grooves configured for winding engagement of the opposite end sections of the first traction body 4100 that have been passed through the first traction channels 1200 and the transition guide channels 2200. Assuming the annular winding grooves have a winding diameter D and the first traction body 4100 has a diameter d, then the distal openings of the two first traction channels 1200 may be spaced apart at a straight-line distance, which is greater than or equal to (D - 3d) and less than or equal to (D + 3d).

Referring to Figs. 63 to 66, in one embodiment, the energy release body 3100 is provided with a cable connecting mechanism 7000, the energy transmission cable 3200 is connected to the cable connecting mechanism 7000. The cable connecting mechanism 7000 may have various implementations. For example, in one embodiment, the cable connecting mechanism 7000 includes a raised adapter block 7100 disposed on the energy release body 3100, and the energy transmission cable 3200 is adapted for winding engagement with the raised adapter block 7100. Alternatively, the cable connecting mechanism 7000 may include an elastic adapter 7200 and an adapter conductor 7300, which are separately provided on the energy release body 3100 and the swing base body 2000. A distal end of the energy transmission cable 3200 may be connected to one of the elastic adapter 7200 and the adapter conductor 7300.

For example, the elastic adapter 7200 may be provided on the energy release body 3100, while the adapter conductor 7300 may be provided on the swing base body 2000. The distal end of the energy transmission cable 3200 may be connected to the adapter conductor 7300, which thereby connects the energy transmission cable 3200 to the elastic adapter 7200. The elastic adapter 7200 is elastically stretchable and contractible, for example, under the action of a spring or the like. In the process of the energy release body 3100 being pivotably assembled with the swing base body 2000, since the elastic adapter 7200 is elastically stretchable and contractible, it always remains in contact with the adapter conductor 7300, creating an electrical connection between them. The swing base body 2000 may also be provided with a seal, which can prevent ingress of any tissue fluid, thereby eliminating the risk of leakage that may be otherwise caused and providing electrical insulation.

Referring to Figs. 67 to 68, in one embodiment, the distal end of the energy transmission cable 3200 is connected to an elastic conductive cable 3300, the elastic conductive cable 3300 is disposed between the distal opening of the first traction channel 1200 and the proximal opening of the transition guide channel 2200. In this way, an electrical connection can be indirectly created between the distal end of the energy transmission cable 3200 and the adapter conductor 7300 by the elastic conductive cable 3300, instead of passing the energy transmission cable 3200 itself through the first traction channel 1200 and the transition guide channel 2200. With this arrangement, during pivoting of the swing base body 2000 relative to the pitch base body 1000, the energy transmission cable 3200 is always absent from the space between the distal opening of the first traction channel 1200 and the proximal opening of the transition guide channel 2200, which is affected by the pivoting of the swing base body 2000 relative to the pitch base body 1000. Accordingly, the energy transmission cable 3200 does not need to deflect any longer and is therefore avoided from a shortened lift span that may otherwise result from the deflection. Such deflection is instead accomplished by the elastic conductive cable 3300, which can arbitrarily stretch or contract between the distal opening of the first traction channel 1200 and the proximal opening of the transition guide channel 2200 while providing desirable electrical conduction due to its elastic deformability.

Referring to Figs. 69 to 72, in one embodiment, the energy release body 3100 includes a first energy delivery jaw 3100a and a second energy delivery jaw 3100b. The first energy delivery jaw 3100a and the second energy delivery jaw 3100b are both assembled on the swing base body 2000 so as to be able to pivot relative to each other to switch between closed and open configurations. Two energy transmission cables 3200 are provided, namely, a first energy transmission cable 3200a and a second energy transmission cable 3200b. The first energy transmission cable 3200a is connected to the first energy delivery jaw, and the second energy transmission cable 3200b is connected to the second energy delivery jaw 3100b. Alternatively, the energy release body 3100 may include more than two energy delivery jaws. In any case, the energy delivery jaws may be configured to move in an interdependent manner. However, the present application is not so limited.

In one embodiment, the pitch base body 1000 defines four first traction channels 1200, and the swing base body 2000 defines two transition guide channels 2200. Each pair of first traction channels 1200 corresponds to a respective one of the transition guide channels 2200. The traction assembly 4000 includes two first traction bodies 4100, which are wound respectively on the first energy delivery jaw 3100a and the second energy delivery jaw and configured to cause them to pivot about respective fixed axes of pivot. Opposite end sections of each first traction body 4100 are threaded through a respective pair of first traction channels 1200. That is, the four end portions of the two first traction bodies 4100 are threaded through the respective four first traction channels 1200. Each first traction body 4100 may be integral, wherein the opposite end sections refer to its two sections extending both from a point, where the traction is fixed to the first 3100a or second energy delivery jaw, to its respective opposite ends. Alternatively, each first traction body 4100 may be composed of two separate component tendons, which are both tied at one end to the first 3100a or second clamping jaw and thus form the first traction body 4100 connected to the first 3100a or second clamping jaw.

In an alternative embodiment, the pitch base body 1000 defines two first traction channels 1200, and the swing base body 2000 defines two transition guide channels 2200. Each of the first traction channels 1200 corresponds to a respective one of the transition guide channels 2200. The traction assembly 4000 includes two first traction bodies 4100, which are wound respectively on the first energy delivery jaw 3100a and the second energy delivery jaw and configured to cause them to pivot about respective fixed axes of pivot. Opposite end sections of each first traction body 4100 are threaded through the two respective first traction channels 1200 in the pitch base body 1000. Therefore, in each first traction channel 1200, there are two end sections of the respective first traction bodies 4100.

When the swing base body 2000 includes a first base portion 2400 and a second base portion 2500, both pivotably coupled to the pitch base body 1000, the distal end face of the first base portion 2400 may define two lateral holes 2400a. The two lateral holes 2400a are configured for passage respectively of the first and second energy transmission cables 3200a, 3200b therethrough, and the second base portion 2500 may define a central channel 2500a in its proximal end face. The first and second base portions 2400, 2500 may be assembled together so that the central channel 2500a is brought into communication with both lateral holes 2400a. Thus, the first and second energy transmission cables 3200a, 3200b can be passed from distal to proximal through the respective two lateral holes 2400a and then together through the central channel 2500a. The central channel 2500a can bundle the first and second energy transmission cables 3200a, 3200b, for example, so that they run side by side parallel to each other in a direction parallel to a pitch direction. This can effectively protect the first and second energy transmission cables 3200a, 3200b from being lengthened or shortened too much in any joint, thus extending their service life.

The various technical features of the foregoing embodiments may be combined in any way. Although not all such combinations have been described above for the sake of brevity, any of them is considered to fall within the scope of this specification as long as there is no contradiction between the technical features. Presented above are merely several embodiments of the present application. Although these embodiments are described with some particularity and in some detail, it should not be construed that they limit the scope of the present application in any sense. It is to be noted that various variations and modifications can be made by those of ordinary skill in the art without departing from the concept of the present application. Accordingly, it is intended that all such variations and modifications are embraced within the scope of this application as defined in the appended claims.

## Claims

1. A clamping instrument tail end, **characterized in that** the clamping instrument tail end comprises:
a pitch base body, wherein a distal end face of the pitch base body is provided with a first contact boss protruding from the distal end face, the pitch base body is provided with first traction channels, and a distal opening of each of the first traction channels is located at the distal end face of the pitch base body; and
a swing base body, wherein a proximal end face of the swing base body is provided with a second contact boss protruding from the proximal end face, the swing base body is pivotably assembled with the pitch base body, such that the first contact boss is in rolling contact with the second contact boss, the swing base body is provided with guide channels, and a proximal opening of each of the guide channels is located at the proximal end face of the swing base body.

2. The clamping instrument tail end according to claim 1, wherein the pitch base body is provided with at least two first traction channels, wherein the guide channels comprise at least two wire guide channels, and wherein a proximal opening of each of the wire guide channels is located at the proximal end face of the swing base body.

3. The clamping instrument tail end according to claim 2, wherein a rolling tangency between the first contact boss and the second contact boss forms a dynamic tangent line of dynamic variable contact, wherein a plane normal to the dynamic tangent line is defined as a dynamic normal plane, wherein a straight line segment connecting the distal opening of one of the first traction channel to the proximal opening of a respective one of the wire guide channels is defined as a line segment of deflection, and wherein an angle between the line segment of deflection and the dynamic normal plane is not greater than 10°.

4. The clamping instrument tail end according to claim 3, wherein the clamping instrument tail end comprises:
a traction assembly, wherein the traction assembly comprises at least one first traction body, each first traction body passes through one of the first traction channels and one of the wire guide channels to connect with a terminal clamping body, and wherein the first traction body has a wire diameter of d, and a distance from any point on the line segment of deflection to the dynamic normal plane is less than 4d.

5. The clamping instrument tail end according to claim 4, wherein at least one of the pitch base body and the swing base body is provided with a motion-restraining mechanism, and wherein the motion-restraining mechanism is configured to restrain the first contact boss to always maintain rolling tangency with the second contact boss.

6. The clamping instrument tail end according to claim 5, wherein the motion-restraining mechanism comprises a first lateral stop member and a second lateral stop member, the first lateral stop member disposed on the pitch base body, the second lateral stop member disposed on the swing base body, the first lateral stop member and the second lateral stop member in restraining contact with each other; and wherein the motion-restraining mechanism further comprises a restraining tooth and a restraining socket, the restraining tooth and the restraining socket are disposed separately on the pitch base body and the swing base body, the restraining tooth and the restraining socket engage with each other.

7. The clamping instrument tail end according to claim 6, wherein the restraining socket is provided in the pitch base body, wherein the pitch base body is provided with two lateral stop protrusions, and wherein the two lateral stop protrusions are disposed on opposite sides of an opening of the restraining socket and are configured to come into restraining contact with sides of the restraining tooth.

8. The clamping instrument tail end according to claim 7, wherein the traction assembly comprises at least two second traction bodies, the pitch base body provided with at least two second traction channels, each of the second traction bodies threaded through one of the second traction channels and connected to the swing base body and configured to drive the swing base body to pivot relative to the pitch base body, wherein the lateral stop protrusions are provided with avoidance recesses, and wherein the avoidance recesses are configured to avoid interference with the second traction bodies.

9. The clamping instrument tail end according to claim 8, wherein all distal openings of the second traction channels are located at the distal end face of the pitch base body and all distal openings of the second traction channels are aligned on a straight line perpendicular to a pivoting direction of the swing base body, and/or wherein the swing base body is provided with at least two traction connecting features, each traction connecting feature corresponding to one of the second traction channels, wherein each of the second traction bodies is connected to the corresponding one of the traction connecting features on the swing base body.

10. The clamping instrument tail end according to claim 9, wherein the pitch base body is provided with two first traction channels, the swing base body is provided with two wire guide channels, each of the first traction channels corresponding to one of the wire guide channels, wherein the traction assembly comprises two first traction bodies, which are wound respectively on a first clamping jaw and a second clamping jaw in the terminal clamping body and configured to cause the first clamping jaw or the second clamping jaw to pivot about a fixed axis of pivot, and a head section and a tail section of each first traction body are threaded through the two first traction channels, or
wherein the pitch base body is provided with four first traction channels, the swing base body is provided with two wire guide channels, each pair of the first traction channels corresponding to one of the wire guide channels, wherein the traction assembly comprises two first traction bodies, which are wound respectively on a first clamping jaw and a second clamping jaw in the terminal clamping body and configured to cause the first clamping jaw or the second clamping jaw to pivot about the fixed axis of pivot, and a head section and a tail section of each first traction body are threaded through a respective pair of the first traction channels.

11. The clamping instrument tail end according to claim 10, wherein at least one of the pitch base body and the swing base body is provided with a guide mechanism, wherein the guide mechanism is configured to guide the winding of the two first traction bodies on the first clamping jaw and the second clamping jaw, wherein the first clamping jaw defines a first annular winding groove, the respective first traction body is wound and connected to the first annular winding groove of the first clamping jaw, and the second clamping jaw is provided with a second annular winding groove, the respective second traction body is wound and connected to the second annular winding groove of the second clamping jaw.

12. The clamping instrument tail end according to claim 11, wherein the guide mechanism comprises a lubricating coating, the lubricating coating provided on inner walls of the wire guide channels, and/or
wherein the guide mechanism comprises a plurality of rolling guide elements, the plurality of rolling guide elements provided on at least one side of at least one of the first traction channels and the wire guide channels, wherein the rolling guide elements are in rolling-guide contact with the first traction bodies, and/or
wherein the guide mechanism comprises a plurality of sliding guide elements, the plurality of sliding guide elements provided on at least one side of at least one of the first traction channels and the wire guide channels, wherein the sliding guide elements are in sliding-guide contact with the first traction bodies.

13. The clamping instrument tail end according to claim 12, wherein an even number of rolling guide elements are provided and paired, wherein the two rolling guide elements of each pair are arranged in symmetry on opposite sides of at least one of the first traction channels and the wire guide channels, or
wherein an even number of sliding guide elements are provided and paired, wherein the two sliding guide elements of each pair are arranged in symmetry on opposite sides of at least one of the first traction channels and the wire guide channels, or
wherein the guide mechanism comprises a plurality of rolling guide elements and a plurality of sliding guide elements, wherein the sum of the number of rolling guide elements and the number of sliding guide elements is an even number, and the rolling and sliding guide elements are paired, the rolling and sliding guide elements of each pair being arranged in symmetry on opposite sides of at least one of the firsttraction channels and the wire guide channels.

14. The clamping instrument tail end according to claim 13, wherein the head section and the tail section of each first traction body are a first stressed wire section and a second stressed wire section, wherein the rolling guide elements are in rolling-guide contact with the first stressed wire sections, and wherein the sliding guide elements are in sliding-guide contact with the second stressed wire sections.

15. An energy instrument tail end, **characterized in that** the energy instrument tail end comprises:
the clamping instrument tail end according to claim 1, wherein the swing base body is pivotably assembled with the pitch base body so as to be able to reciprocally along a predefined direction, and the guide channels comprise transition guide channels; and
an energy component comprising an energy release body and an energy transmission cable, the energy release body assembled on the swing base body, the energy transmission cable passes through one of the first traction channels and one of the transition guide channels and connected to the energy release body.

16. The energy instrument tail end according to claim 15, wherein the energy instrument tail end comprises:
a traction assembly comprising a first traction body, wherein there are at least two first traction channels and at least two transition guide channels; the energy release body is pivotably connected to the swing base body; the first traction body passes through one of the first traction channels and the transition guide channels, connected to the energy release body and configured to cause the energy release body to reciprocally pivot relative to the swing base body along a predefined direction.

17. The energy instrument tail end according to claim 15, wherein the pitch base body defines two or three first traction channels, the swing base body correspondingly provided with two or three transition guide channels, each of the first traction channels corresponding to a respective one of the transition guide channels, wherein the first traction body is wound on the energy release body and configured to cause the energy release body to pivot about a fixed axis of pivot, wherein a head section and a tail section of the first traction body are threaded through different ones of the first traction channels and different ones of the transition guide channels, the energy transmission cable passing through one of the first traction channels and one of the transition guide channels, or
wherein a head section and a tail section of the first traction body are threaded through respective two of the first traction channels and respective two of the transition guide channels, the energy transmission cable passing through a different one of the first traction channels and a different one of the transition guide channels.

18. The energy instrument tail end according to claim 17, wherein the first traction channel that the energy transmission cable is threaded through has a curved transition surface, and/or
wherein a cable separation component is provided between the first traction channel that the energy transmission cable is threaded through and the two first traction channels that the first traction body is threaded through to separate the energy transmission cable from the adjacent first traction body.

19. The energy instrument tail end according to claim 18, wherein at least one of the pitch base body and the swing base body is provided with a movement guide mechanism, wherein the movement guide mechanism comprises a plurality of rolling guide elements, the plurality of rolling guide elements located on at least one side of at least one of the first traction channels and the transition guide channels, wherein the rolling guide elements is in rolling-guided contact with the first traction body for guiding the first traction body to be wound on the energy release body.

20. The energy instrument tail end according to claim 19, wherein the energy release body defines a annular winding groove, the first traction body is wound and connected to the annular winding groove, and the annular winding groove has a winding diameter of D, wherein the first traction body has a diameter of d, and distal openings of two of the first traction channels is spaced apart at a straight-line distance, which is greater than or equal to (D - 3d) and less than or equal to (D + 3d).

21. The energy instrument tail end according to claim 15, wherein the energy release body is provided with a cable connecting mechanism, and wherein the energy transmission cable is connected to the cable connection mechanism.

22. The energy instrument tail end according to claim 21, wherein the cable connecting mechanism comprises a raised adapter block disposed on the energy release body, the energy transmission cable is wound and connected to the raised adapter block, and/or
wherein the cable connecting mechanism comprises an elastic adapter and an adapter conductor, which are provided separately on the energy release body and the swing base body, wherein the energy release body is pivotably assembled with the swing base body so that the elastic adapter and the adapter conductor are elastically brought into electrical contact with each other, and a distal end of the energy transmission cable is connected to one of the elastic adapter and the adapter conductor.

23. The energy instrument tail end according to claim 22, wherein the distal end of the energy transmission cable is connected to an elastic conductive cable, the elastic conductive cable located between a distal opening of the first traction channel and a proximal opening of the transition guide channel, and wherein the distal end of the energy transmission cable is indirectly electrically connected to the adapter conductor via the elastic conductive cable.

24. The energy instrument tail end according to claim 15, wherein the energy release body comprises a first energy delivery jaw and a second energy delivery jaw, which are assembled on the swing base body so as to be able to pivot relative to each other to switch between a closed configuration and an open configuration, wherein energy transmission cables are provided, which include a first energy transmission cable and a second energy transmission cable, the first energy transmission cable connected to the first energy delivery jaw, and the second energy transmission cable connected to the second energy delivery jaw; the swing base body comprises a first base portion and a second base portion, which are both pivotably connected to the pitch base body, the second contact boss provided on the second base portion, a distal end face of the first base portion provided with two lateral holes, a proximal end face the second base portion provided with a central channel, the central channel brought in communication with the two lateral holes so that the first energy transmission cable and the second energy transmission cable are able to be passed through the two lateral holes respectively and then together out through the central channel.

25. A surgical instrument, **characterized in that** the surgical instrument comprises:
the clamping instrument tail end of any one of claims 1 to 14, or the energy instrument tail end of any one of claims 15 to 24.
